# EUROPEAN PATENT SPECIFICATION

(11) **EP 0 655 225 B1**
(45) Date of publication and mention of the grant of the patent: **08.03.2000**
(21) Application number: 94202749.1
(22) Date of filing: 22.09.1994
(51) Int. Cl.: A61B 18/02

(54) **Cryo-ablation catheter**
Katheter zur Kryo-Abtragung
Cathéter d'ablation cryogénique

(30) Priority: 26.10.1993 NL 9301851
(43) Date of publication of application: 31.05.1995
(73) Proprietor: CORDIS EUROPA N.V., NL-9301 LJ Roden (NL)
(72) Inventor: Wijkamp, Arnoldus Cornelius Johannes, NL-9302 AW Roden (NL)
(74) Representative: 't Jong, Bastiaan Jacobus

(56) References cited:
- EP-A- 0 395 307
- EP-A- 0 437 377
- DE-A- 2 332 513
- DE-A- 2 435 443
- DE-B- 1 466 790
- FR-A- 1 605 386
- GB-A- 2 226 497
- KUCHLING: "Taschenbuch der Physik" 5, , VERLAG HARRI DEUTSCH, THUN U. FRANKFURT/MAIN

## Description

The invention relates to a cryo-ablation catheter as known from DE-A-2 332 513.

The catheter known from this publication comprises a tube-like basic body with a closed heat, made of thermally conductive material, at the distal end. A pressure line of pliable synthetic material has been received in the basic body comprising a nozzle at its end close to the head. A refrigerant having a low temperature is sucked through the pressure line forming a thermal insulation for the refrigerant. The head is cooled by the contact with the refrigerant and also because of the evaporation thereof. This cooling effect based on evaporation is known as the Joule-Thompson effect. With the head, thus cooled down, ablation procedures can be carried out inside organs, for instance the stomach of a patient.

The invention has for its object to improve a catheter of the type mentioned above, such that it can be manufactured with a small diameter so that ablation procedures can be carried out for instance inside the heart of a patient. The catheter of the invention has to be properly pliable and despite a small diameter an appropriate, very low temperature of the head of the catheter must be obtainable.

This aim is achieved with the catheter as characterized in claim 1. The fluid under pressure, which can be a liquid as well as a gas, is pre-cooled to a low value. Due to the pressure, sufficient fluid can be supplied to the distal end of the catheter and a substantial pressure drop can be obtained at the restriction, so that at the tip of the catheter a very low temperature can be achieved due to the Joule-Thompson effect already referred to. Even with small diameters of the pressure line, having as a consequence a relatively large surface area of the pressure line in relation to the cross-sectional area thereof, only little heat transfer will take place across the wall of the pressure line, in view of the returning stream of expanded gas flowing through the catheter. Inside the cooling means the fluid is cooled to a temperature of for instance - 40°C.

A suitable embodiment of this device is characterized in claim 2. The discharged expanded refrigerant can still have such a low temperature at the proximal end of the discharge channel that it can be used to pre-cool the refrigerant in the pressure line in a suitable manner.

Another suitable, preferred embodiment is characterized in claim 3. Thus the fluid in the pressure line can be cooled considerably and very simply, wherein the cooling means need to take up only a relatively small volume. As the refrigerant under pressure is already present anyway, pre-cooling can be achieved in a very efficient manner.

The measure as set out in claim 4 is preferably employed. Because of this the temperature of the fluid to be supplied into the pressure line in the catheter can be monitored carefully, so that the desired cooling effect at the tip of the catheter can be achieved for certain.

When employing the measure as set out in claim 5, the idea of partly embodying the pressure line as heat exchanger is abandoned completely and consequently also the additional pre-cooling effect of the returning stream of expanded gas is relinquished entirely. In spite of this it is possible to achieve a desired very low temperature of the catheter tip. By extending the pressure line and the discharge channel axially along their entire length, the basic body can have a small diameter which is conducive to treatment to be carried out with the catheter. To achieve an adequate discharge of the expanded refrigerant and consequently, to optimize the cooling effect at the tip, the discharge channel could be connected to exhaust means, so that a sufficient pressure difference at the restriction at the end of the pressure line can be maintained.

An advantageous embodiment is characterized in claim 6. Inside the lumen of the basic body, the pressure line is entirely surrounded by the stream of expanded refrigerant, so that minimal heat transfer via the wall of the catheter to the pressure line can take place.

To properly monitor the functioning of the catheter, the measure as set out in claim 7 is preferably employed. A thermistor may suitably be used as temperature sensor. Its signal lines can be made of very thin wires conducting electricity which will reduce the free cross-section of the lumen minimally.

The invention will be explained below with reference to a description of an example of an embodiment.
Fig. 1 illustrates schematically a cryo-ablation catheter means according to the invention.
Fig. 2 shows a cross-section of the distal end-section of the actual catheter in fig. 1 indicated with arrow II.
Fig. 3 shows a cross-section at arrows III-III in fig. 2.
Fig. 4 represents a partly cross-sectional view at the Y-piece indicated with arrow IV in fig. 1.

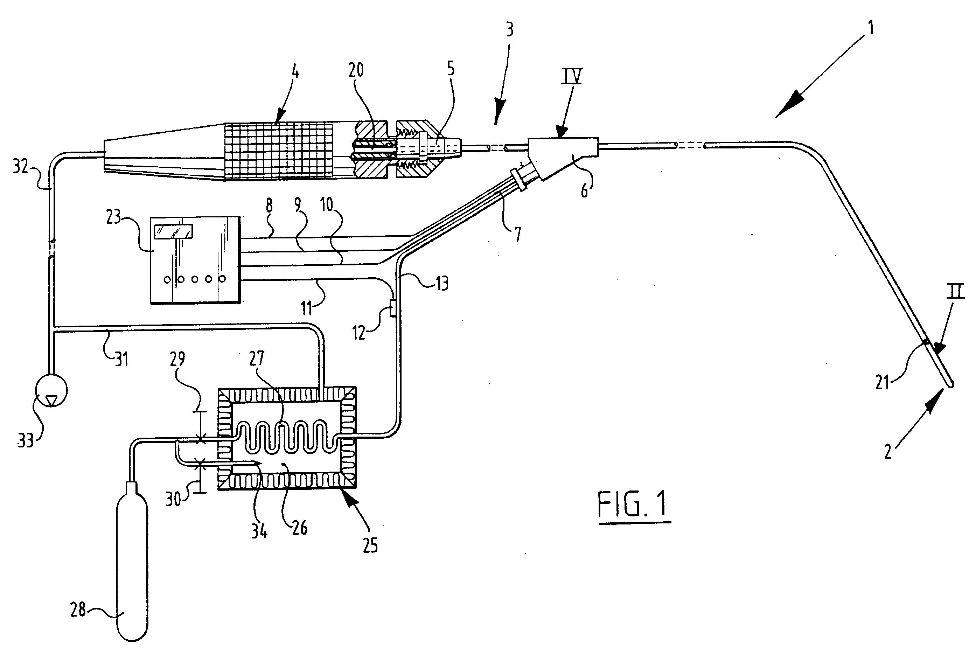

The catheter means in fig. 1 shows a catheter 1 according to the invention with a distal end 2 and a proximal end 3. The proximal end 3 carries a connecting member 5, by means of which the catheter has been received in a handle 4. The catheter 1 may be for single use, whereas the handle 4 is reusable.

The catheter 1 comprises a basic body 15 with at the distal end 2 a closed head 14 made of a thermally conductive material, for instance a metal.

The basic body 15 comprises one lumen 20 which serves as discharge channel in a manner to be explained later.

Inside the lumen 20 a pressure line 13 has been received, extending from the proximal end 3 of the catheter 1 to the distal end 2. By means of bonding agent 16 the pressure line 13 has been secured in the head 14. During the manufacturing process the distal end of the pressure line 13 is first secured in the head 14, after which the basic body 15 is pushed over the appropriate section of the head 14 and fixed to it.

The pressure line 13 comprises a restriction 17 at its distal end inside the head 14.

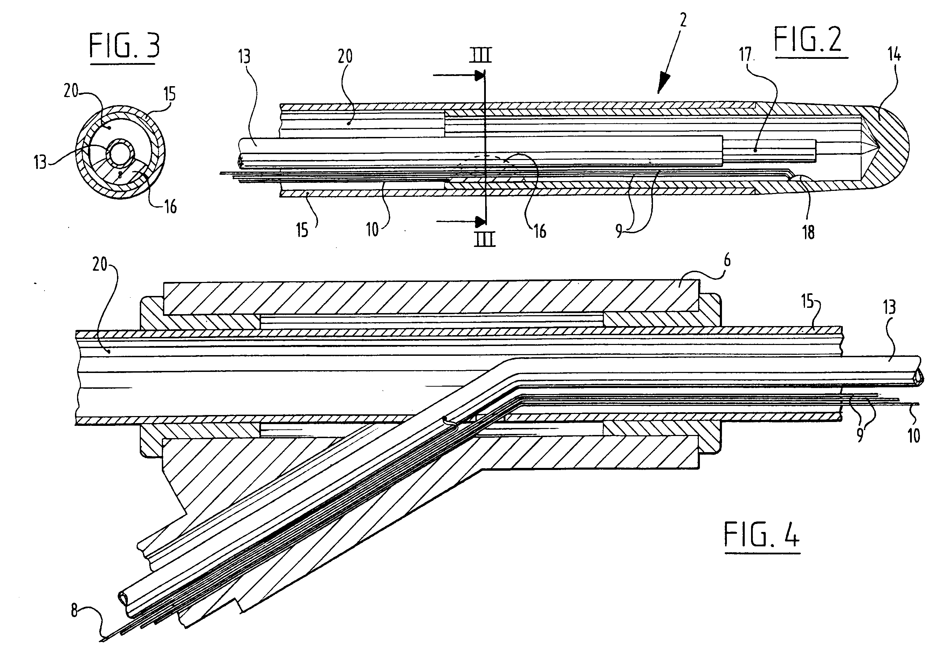

As fig. 4 illustrates, the pressure line 13 is led outside the basic body at a Y-piece 6 in the catheter 1. The pressure line 13 and the signal lines 8 - 10 still to be described, are led outside in the Y-piece 6 in a sealed manner so that the discharge channel formed by the lumen 20 remains separate.

Via the pressure line 13, refrigerant under high pressure can be conveyed to the distal end of the catheter. After passing the restriction 17 this refrigerant will expand, drawing heat from the surroundings. Because of this the head 14 will be cooled to a very low temperature.

The expanded gaseous fluid returns via the discharge channel 20 formed by the lumen, to the proximal end 3 of the catheter. Inside the handle 4 the discharge channel 20 is, sealed in an appropriate manner, connected to a line 32 which discharges the expanded fluid subsequently. A pump 33 may be received in this line 32, as is the case in the illustrated example of the embodiment, in order to ensure that also in case of very small diameters of the catheter 1, the expanded gas is discharged properly and that a sufficient pressure difference is maintained at the restriction 17 in order to achieve the desired cooling effect.

According to the invention the pressure line 13 is made of a synthetic material with a, compared to metal, low modulus of elasticity and high thermal resistance coefficients. The catheter 1 and in particular its distal end 2 can be made adequately pliable because of the low modulus of elasticity of the material of which the pressure line 13 has been made.

To achieve an adequate cooling effect in the head 14 of the catheter, the refrigerant is pre-cooled in the cooling means 25 prior to it being conveyed to the pressure line. The cooling means illustrated schematically in fig. 1 comprise an isolated cooling chamber 26, through which a tube 27 extends helically. The pressure line is connected to this tube 27. From a source of refrigerant , here depicted in the form of a pressure cylinder 28, a pressure fluid is supplied to the pressure line 27. By means of an adjustable valve 29, the required quantity can be set.

In front of the valve 29 a line branches off from the refrigerant line which , via a restriction 34, debouches in the cooling chamber 26. The quantity of fluid supplied into the cooling chamber 26 is set by means of the control valve 30. When passing the restriction 34 the refrigerant expands inside the chamber 26 and on doing so draws heat from the surroundings, that is to say from the refrigerant passing through the tube 27 which consequently will be cooled. The expanded fluid is extracted from the chamber 26 by the line 31, so that a sufficient pressure difference is maintained across the restriction.

As fig. 1 illustrates schematically, a temperature sensor 12 has been arranged at the proximal end of the pressure line, which , via a signal line 11, is connected with measuring equipment 23. Thus the temperature of the refrigerant, supplied into the proximal end of the pressure line 13, can be checked. On the basis of the measured temperature, the control valve 30 can be set. In another embodiment the control valve 30 can be operated by a control means on the basis of the temperature as measured with the sensor 12.

A temperature sensor 18 has also been received in the head 14 of the catheter. This sensor is connected with measuring equipment 20 via signal lines 9. With the aid of the temperature sensor 18, the temperature of the head 14 of the catheter can be read off. The measured value can, if so desired, also be used to set the control valve 29. With another embodiment, operating the control valve 29 can be done automatically in accordance with the temperature measured in the head 14.

At the distal end the catheter is provided with an annular electrode which is also connected to measuring equipment 23 by means of a signal line 10. By means of the annular electrode 21 in combination with the electrically conductive head 24, measurements can be taken inside organs in order to determine the correct position for carrying out the ablation procedure.

The catheter means according to the invention is for instance used for ablating surface tissue inside the heart when treating certain cardiac arrhythmias. By cooling the tissue to a great extent, it will be frozen locally and be destroyed.

In the case of the illustrated catheter the reinforcing layer of the basic body made of braided metal wires forms a conductor for measuring signals, and the signal line 8 is therefore connected to this reinforcing layer at the Y-piece 6.

Due to the relatively high thermal resistance coefficient of the material of which the pressure line 13 has been made, the pre-cooled fluid will, at the most, absorb only little heat from the surroundings. Inside the basic body 15 of the catheter, the pressure line 13 extends through the central lumen. The expanded gas which is being discharged from the head 14, passes through this lumen. Initially this expanded gas has a very low temperature and is heated only very slightly in the head. The gas passing through the discharge channel 20 still has therefore a low temperature, so that consequently also no or only little warming up of the refrigerant supplied under pressure, will occur.

Although this has not been illustrated in fig. 1, the section of the pressure line 13 connected to the cooling means 25 will, as a rule, be provided with an isolation layer in order to prevent also here warming up of the pressure fluid.

It should be noted that in the figures only a conceivable embodiment is shown. Other embodiments are possible. The cooling means 25 for instance could be received in the handle 4. In that case the cooling line 13 can be surrounded by expanded exhaust fluid over almost its entire length, so that the temperature of the pressure fluid becomes properly controllable.

As has been mentioned before, with certain embodiments and certain settings of the fluid streams the expanded fluid flowing back can still have such a low temperature at the proximal end 3, that it can be used in the cooling means close to the proximal end to pre-cool the pressure fluid.

All these conceivable variants are considered to be included in the invention.

## Claims

1. Cryo-ablation catheter (1) means , comprising a tube-like basic body (15) with a proximal (3) and a distal end (2), wherein at the distal end (2) a closed head (14) made of a thermally conductive material has been arranged, a pressure line (13) extending in the basic body (15) from close to the proximal end (3) to close to the head (14) comprising a nozzle (17) at its distal end, and a discharge channel (20) extending from the head (14) to the proximal end (3), wherein the pressure line (13) is made of a pliable synthetic material and the pressure line (13) is, at the proximal end (3), connected with a source (28) of fluid under pressure characterized in that the nozzle is a restriction (17) and that at the proximal end (3) cooling means (25) are provided for the purpose of cooling the fluid.

2. Means as claimed in claim 1, wherein the cooling means comprise a heat exchanger which is connected to the pressure line on one side and to the discharge channel on the other side.

3. Means as claimed in claim 1 or 2, wherein the cooling means (25) comprise an expansion-cooler in which part of the fluid under pressure expands whilst drawing heat from the remainder of the fluid.

4. Means as claimed in one of the claims 1-3, wherein a temperature sensor (12) has been arranged at the proximal end of the pressure line (13).

5. Means as claimed in claim 1, wherein the pressure line (13) and the discharge channel (20) extend axially over their entire length in the basic body (15).

6. Means as claimed in claim 1, wherein the discharge channel is formed by a lumen (20) of the basic body (15) and the pressure line (13) is a separate line, received, with an interstice, in this lumen (20).

7. Means as claimed in one of the previous claims, wherein a temperature sensor has been arranged in the head and of which signal lines extend through the discharge channel to the proximal end of the catheter.

## Patentansprüche

1. Kryo-Ablationskatheter-Einrichtung (1) mit einem röhrenförmigen Grundkörper (15) mit einem proximalen Ende (3) und einem distalen Ende (2), wobei an dem distalen Ende (2) ein geschlossener Kopf (14) aus einem thermisch leitfähigen Material angeordnet ist, einer Druckleitung (13), die in dem Grundkörper (15) von einer nahe dem proximalen Ende (3) gelegenen Stelle zu einer nahe dem Kopf (14) gelegenen Stelle verläuft und die an ihrem distalen Ende eine Düse (17) aufweist, sowie einem Ausflußkanal (20), der von dem Kopf (14) zu dem proximalen Ende (3) verläuft, wobei die Druckleitung (13) aus einem biegsamen synthetischen Material hergestellt und an dem proximalen Ende (3) an eine Quelle (28) für Druckfluid angeschlossen ist, dadurch gekennzeichnet, daß die Düse eine Verengung (17) ist, und daß an dem proximalen Ende (3) eine Kühleinrichtung (25) vorgesehen ist, mit der sich das Fluid kühlen läßt.

2. Einrichtung nach Anspruch 1, wobei die Kühleinrichtung einen Wärmetauscher aufweist, der auf einer Seite mit der Druckleitung und auf der anderen Seite mit dem Ausflußkanal verbunden ist.

3. Einrichtung nach Anspruch 1 oder 2, wobei die Kühleinrichtung (25) einen Expansionskühler aufweist, in dem sich ein Teil des Druckfluids ausdehnt, während dem übrigen Teil des Fluids Wärme entzogen wird.

4. Einrichtung nach einem der Ansprüche 1 bis 3, wobei an dem proximalen Ende der Druckleitung (13) ein Temperatursensor (12) angeordnet ist.

5. Einrichtung nach Anspruch 1, wobei die Druckleitung (13) und der Ausflußkanal (20) axial über ihre gesamte Länge hinweg in dem Grundkörper (15) verlaufen.

6. Einrichtung nach Anspruch 1, wobei der Ausflußkanal von einem Lumen (20) des Grundkörpers (15) gebildet wird und die Druckleitung (13) eine separate Leitung ist, die mit einem Zwischenraum in dem Lumen (20) aufgenommen ist.

7. Einrichtung nach irgendeinem der vorgenannten Ansprüche, wobei ein Temperatursensor in dem Kopf angeordnet ist und Signalleitungen desselben durch den Ausflußkanal zu dem proximalen Ende des Katheters verlaufen.

## Revendications

1. Moyens formant cathéter d'ablation cryogénique (1), comprenant un corps de base en forme de tube (15) avec une extrémité proximale (3) et une extrémité distale (2), dans lesquels il est prévu, à l'extrémité distale (2) une tête fermée (14) réalisée en matériau thermiquement conducteur, une ligne de pression (13) s'étendant dans le corps de base (15) depuis les environs de l'extrémité proximale (3) jusqu'aux environs de la tête (14) comprenant une buse (17) à son extrémité distale, et un canal de décharge (20) s'étendant de la tête (14) à l'extrémité proximale (3), dans lesquels la ligne de pression (13) est réalisée en matériau synthétique pliable et la ligne de pression (13) est, à l'extrémité proximale (3), connectée à une source (28) de fluide sous pression, caractérisés en ce que la buse est un rétrécissement (17) et en ce qu'il est prévu, à l'extrémité proximale (3), des moyens de refroidissement (25) pour refroidir le fluide.

2. Moyens selon la revendication 1, dans lesquels les moyens de refroidissement comprennent un échangeur de chaleur qui est d'un côté connecté à la ligne de pression et de l'autre au canal de décharge.

3. Moyens selon la revendication 1 ou 2, dans lesquels les moyens de refroidissement (25) comprennent un appareil de refroidissement-détente dans lequel une partie du fluide sous pression se détend en absorbant la chaleur du restant du fluide.

4. Moyens selon l'une quelconque des revendications 1-3, dans lesquels un capteur de température (12) est prévu à l'extrémité proximale de la ligne de pression (13).

5. Moyens selon la revendication 1, dans lesquels la ligne de pression (13) et le canal de décharge (20) s'étendent suivant l'axe sur toute leur longueur dans le corps de base (15).

6. Moyens selon la revendication 1, dans lesquels le canal de décharge est formé par une lumière (20) du corps de base (15) et la ligne de pression (13) est une ligne indépendante, logée, avec un interstice, dans cette lumière (20).

7. Moyens selon l'une quelconque des revendications précédentes, dans lesquels un capteur de température est prévu dans la tête et dont les lignes de signal s'étendent à travers le canal de décharge jusqu'à l'extrémité proximale du cathéter.
